# EUROPEAN PATENT APPLICATION

(11) **EP 1 262 144 A1**
(43) Date of publication of application: **04.12.2002**
(21) Application number: 02077082.2
(22) Date of filing: 27.05.2002
(51) Int. Cl.: A61B 5/00

(54) **Spread spectrum telemetry of physiological signals**

(30) Priority: 25.05.2001 US 864213
(71) Applicant: CME Telemetrix Inc., Waterloo, Ontario N2L 5Z4 (CA)
(72) Inventor: Cadell, Theodore C., Waterlo, Ontario N2K 2C2 (CA); Metzger, Dennis, Kitchener, Ontario N2N 1C7 (CA)
(74) Representative: Loven, Keith James

(57) **Abstract**

The invention disclosed is an apparatus, or system, and methodology for power efficient, flexible, data efficient wireless transmission, receipt and interpretation of signals from a patient, such signals reflecting one or more measured physiological and patient specific parameters such as an electrocardiogram, electroencephalogram, electromylogram and/or patient ID. The system includes a mobile transmitter for attachment to a patient, which is a battery powered sensor/transmitter device for transmission of enhanced data transmission rate signals in multiple frequencies within a given frequency band; a receiver for receiving the signals; and a display analysis and/or recording device for interpretation of the received signals. The system operates using a spread spectrum transmission technique which reduces interference with the detection of the transmitted signals. The mobile transmitter and the receiver include corresponding optical components for establishing a duplex optical link allowing for changes to operating characteristics while transmission is occurring.

## Description

### Field of the Invention

The present invention relates to the field of telemetry used in applications where it is advantageous to monitor signals, more specifically, in monitoring the physiological signals of a patient, using in particular spread spectrum transmissions.

### Description of the Prior Art

Telemetry systems are well known in the field of physiological monitoring. For a number of years systems that transmit a plurality of patient signals such as electro cardiograms (ECG), or electroencephalogram (EEG) signals, without wires have been known. The advantages of such systems is obvious insofar as patients are allowed freedom of movement, being unhampered by connecting wires between monitors and sensing devices which are attached to a patient. Such systems allow for ambulation of a patient so that the signals are transmitted from a unit worn by the patient to a central monitoring unit such as a nurses' station.

In certain medical facilities, usually in intensive care units, transmitters located at a patient's bedside are used to transmit signals from patients, who are being monitored for ECG signals, blood pressure, respiration rates, pulse rates, etc. The transmission of these signals is to a nurses' station where incoming signals are monitored. A number of patients may be monitored in this way and software driven alarms may be used to alert the care professional's attention when one or more of the monitored signals is of concern.

In another setting of physiological signal monitoring, telemetry devices have not been used. This setting is in respect of stress tests. These tests monitor the electrical activity of the heart of a patient who is wearing sensor leads which are still routinely attached by cables to a monitor. Introduction of a telemetry-based system would have obvious advantages however current devices are incapable of solving this problem.

Early telemetry systems incorporated FM/FM analogue modulation, however these systems were often susceptible to a large amount of DC drift which increases the likelihood of a false alarm. Such systems also suffer from fairly inefficient use of available bandwidth which limits the number of channels that can be transmitted and the frequency responses of the transmitted channels. An approach to solving these problems has been the conversion of analogue signals to digital signals, see for examples U.S. 5,205,294 issued to Flach et al. and U.S. 4, 958, 645 issued to Cadell et al. Where telemetry devices are used, the systems operate by a variety of digital modulation schemes which modulate the RF carrier. Unfortunately these systems are limited to a limited number of signals due to channel and bandwidth restrictions. In addition, these systems are subject to interference and noise from competing signals, and are thus less than ideal in performance.

In an attempt to overcome some of these limitations Burrows (U.S. 5,381,798) described a device which uses spread spectrum technology to transmit physiological data from a patient to a monitoring station. However, the system taught by Burrows suffers from a number of drawbacks. For example, it uses frequency shift keying modulation which limits the data transmission rate in a given bandwidth. Consequently, in the event of adaptation of the system for monitoring a larger number of patient parameters, the Burrows system as disclosed is inadequate. Furthermore, with the Burrows system changes to characteristics of the data scrambler and frequency spreader would only be possible when the transmitter is off line. Finally, because of the over sampling and DSP design taught by Burrows, the device described would be impractical for use as a battery powered device. Too much power would be required thereby resulting in frequent recharging of the device, and in any event, the physical size limitations of the components coupled with the power consumption required to achieve the system taught by Burrows would dictate a battery size that would be impractical. In the light of these limitations a more power efficient, flexible, data efficient device is required.

### Summary of the Invention

The present invention overcomes the aforementioned deficiencies in the field by providing a method and a system which provides more power efficient, flexible, data efficient wireless transmissions.

In accordance with an aspect of the present invention there is provided a mobile transmitter for monitoring a patient via physiological data comprising: a sensor interface for coupling to sensors disposed on the patient for collecting physiological data therefrom; a digital controller having an input for analogue data from the sensor interface, an output for serial digital data derived from analogue data and an optical receiver and transmitter for establishing a bi-direction optical link for receiving mobile transmitter configuration data during operation of the mobile transmitter; and a radio frequency transmitter for radio transmission of the serial digital data in dependence upon stored mobile transmitter configuration data.

In accordance with another aspect of the present invention there is provided base station for monitoring a patient via physiological data comprising: an antenna array for receiving the wireless transmission from the mobile transmitter; a receiver including an input coupled to the antenna array, an interface having an output for digital data derived from the radio transmission and an input of mobile transmitter configuration data, and an optical receiver and transmitter for establishing a bi-direction optical link for transmitting mobile transmitter configuration data to an adjacent mobile transmitter; and a monitor coupled to the interface for display of the physiological data and for effecting transfer of mobile transmitter configuration data via the bi-directional optical link during operation of the mobile transmitter.

In accordance with a further aspect of the present invention there is provided a system for monitoring a patient via physiological data comprising: a mobile transmitter including a sensor interface for coupling to sensors disposed on the patient for collecting physiological data therefrom, a digital controller having an input for analogue data from the sensor interface, an output for serial digital data derived from analogue data and an optical receiver and transmitter for establishing a bi-direction optical link for receiving mobile transmitter configuration data, and a radio frequency transmitter for radio transmission of the serial digital data in dependence upon stored mobile transmitter configuration data; and a base station including an antenna array for receiving the wireless transmission from the mobile transmitter, a receiver including an input coupled to the antenna array, an interface having an output for digital data derived from the radio transmission and an input of mobile transmitter configuration data, and an optical receiver and transmitter for establishing a bi-direction optical link for transmitting mobile transmitter configuration data to an adjacent mobile transmitter, and a monitor coupled to the interface for display of the physiological data and for effecting transfer of mobile transmitter configuration data via the bi-directional optical link during operation of the mobile transmitter.

The present invention provides a more data efficient telemetry system by providing a method to achieve a greater data transmission rate in a smaller bandwidth, within the system which receives physiological signals from a patient and then translates the signals into a format which is suitable for transmission using a spread spectrum signal. The data contained in the spread spectrum signal is then decoded and reformatted. The reformatted physiological signal is subsequently displayed, recorded, printed, analysed or otherwise processed.

The present invention also provides a method of providing multiple frequencies of transmission within the 902 to 928 MHz band of frequencies for the simultaneous transmission of an increased number of signals.

The present invention is a battery operated system which requires infrequent operational charging that receives physiological signals from a patient and then translates the signals into a format which is suitable for transmission using a spread spectrum signal. The data contained in the spread spectrum signal is then decoded and reformatted. The reformatted physiological signal is subsequently displayed, recorded, printed, analysed or otherwise processed. The use of battery power provides the patient with increased movement flexibility. To meet the demand of 24 hours between battery changes and to limit the size of the equipment, i.e., the transmitter, a comprehensive design change was undertaken to reduce the current and limit the size.

The present invention also provides a system that receives physiological signals from a patient and then translates the signals into a format which is suitable for transmission using a spread spectrum signal and allows for changes during operation of the characteristics of scrambling, digital sequence code, frequency and data rate. The data contained in the spread spectrum signal is then decoded and reformatted. The reformatted physiological signal is subsequently displayed, recorded, printed, analysed or otherwise processed.

In a preferred embodiment of the invention there is provided a system for the transmission of physiological signals from a patient to a data receiving device which includes an acquisition system for the detection of desired physiological data from a patient and for processing an analogue signal which corresponds to the data. The system also includes an analogue to digital conversion device which is operatively associated with the data acquisition system. This device is for converting the analogue signals which correspond to the physiological data into a serial digital data stream. The serial digital data stream is combined by a transmission device with a digital code sequence to form a combined transmission signal which is transmitted by the transmission device via spread spectrum transmission over a wide frequency bandwidth. The combined signal is received by a receiving device and a data signal demodulation device separates the serial digital data stream from the digital code sequence. The serial digital data stream from the demodulating device is then processed by a data reformatting processor and the output of a physiological data display, recording and/or analysis device for the receipt of the output from said reformatting processor.

In a preferred embodiment of the invention, the system is a patient monitor telemetry device for the acquisition of data of a design which utilizes spread spectrum radio frequency (RF) technology to increase data integrity and range. In particular, the system makes use any one of the well known bands used in spread spectrum transmissions, namely the 902-928 MHz band, the 2.4-2.5 GHz, or the 5.725-5.785 GHz industrial, scientific, and medical (ISM) band which allows for unlicensed operation in most countries. In a preferred embodiment the system incorporates a transmitter using a frequency synthesizer thereby providing multiple frequencies of transmission within the 902-928 MHz band.

In yet a further preferred embodiment, the system of the invention is incorporated into a modular design to facilitate adaptation to a number of applications and changing requirements. The system has a high data throughput.

In a further embodiment differential quadrature phase shift keying (DQPSK) modulation is utilized to reduce the serial data stream bit rate thereby increasing the data transmission rate for an equivalent bandwidth.

In yet a further embodiment, the remote part of the system is energy efficient thereby allowing for extensive battery life (in a preferred embodiment up to 24 hours) and as such requiring infrequent operational recharging.

In practice the system of this invention can allow for multiple patient transmitters (in the three ISM bands depending upon the particular bandwidth can be up to 100 or more) operating simultaneously in the same location. The sensor/monitor and transmitter of the system is small and light-weight and is easily adapted to functional requirements. The system of this invention is also platform independent having a high speed direct memory access (DMA) computer interface to central monitoring stations.

### Brief Description of the Drawings

FIG. 1 illustrates in a block diagram, of a patient monitoring system in accordance with an embodiment of the present invention;
FIG. 2 illustrates in a block diagram, detail of the mobile transmitter of FIG. 1;
FIG. 3 illustrates in a block diagram, detail of the receiver of FIG. 1;
FIG. 4 illustrates in a block diagram, detail of the RF receiver of FIG. 3;
FIG. 5 illustrates in a block diagram, detail of the despreader of FIG. 3;
FIG. 6 illustrates in a block diagram, details of the digital receiver and optical transmitter/receiver of FIG. 3;
FIG. 7 illustrates in a block diagram, further details of the mobile transmitter of Figs. 1 and 2; and
FIG. 8a, 8b, and 8c graphically illustrate optical signals exchanged between the digital receiver of Fig. 3 and the mobile transmitter of Fig. 2.

### Detailed Description of the Preferred Embodiments

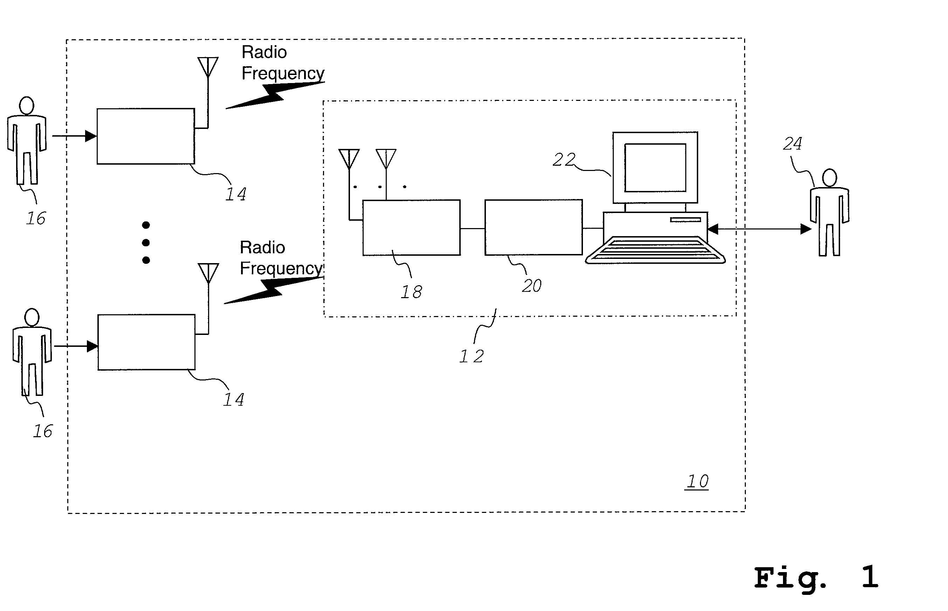

Referring to Fig. 1, there is illustrated, in a block diagram a patient monitoring system in accordance with an embodiment of the present invention. The patient monitoring system 10 includes a base station 12 and a plurality of mobile transmitters 14 coupled to sensors disposed on patients 16. The base station 10 includes an antenna network 18, a receiver 20, and a monitor or computer 22. The monitor 22 allows an attendant 24 to view data for patients 16.

Fig. 1 provides a general overview of a preferred embodiment of the invention. It can be particularized to specific settings and uses for monitoring physiological signals such as electrocardiogram (ECG), electroencephalogram (EEG) or electromylogram (EMG). While the description detailed below is concerned with an embodiment used to monitor EEG, it is to be understood that the present invention can be applied to any setting where remote monitoring of physiological systems is desired or required.

In operation, patients 16 are connected by sensors to the mobile transmitter 14. A physiological signal of interest from the patient is transmitted by spread spectrum techniques to the antenna network 18 that provides the signal to the receiver 20. The receiver 20 provides output that can be directed to the monitor 22, and the data analysis or image creation is conducted with or without input by the attendant 24. The remaining figures detail an example of an embodiment of an EEG system that is one particularization of the general system of Fig. 1.

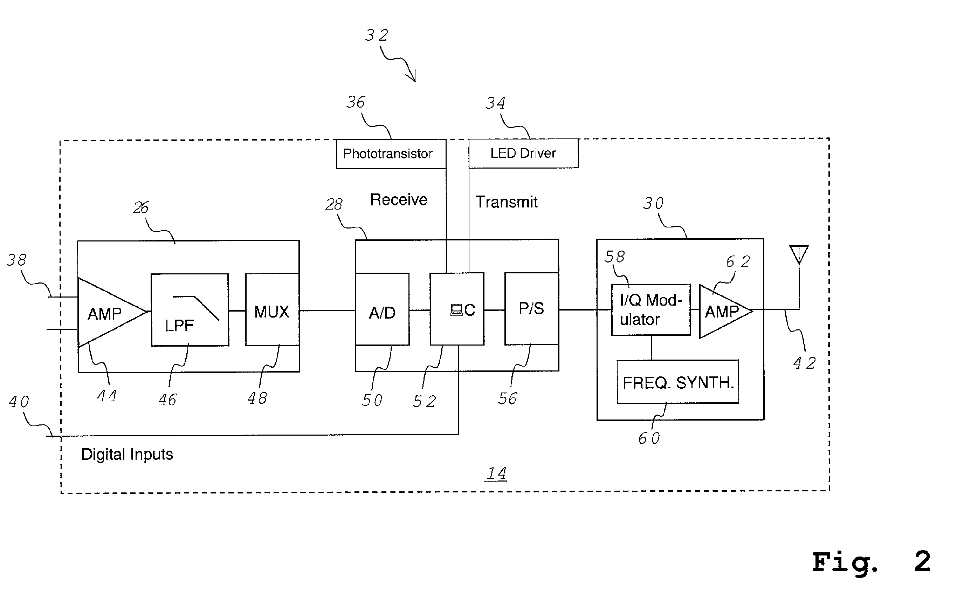

Referring to Fig. 2, there is illustrated in a block diagram detail of the mobile transmitter of Fig. 1. The mobile transmitter 14 includes three functional modules, namely a sensor interface/analogue controller 26, digital processor/controller 28, and an RF transmitter 30. The sensor interface 26 includes connector electrodes 38 and a channel analogue amplifier/filter module having an amplifier (AMP) 44, a low pass filter (LPF) 46 and a multiplexer (MUX) 48. The digital processor/controller 28 includes an analogue to digital converter (A/D) 50, a microcontroller (:C) 52 and a formatter parallel to serial (P/S) 56. A full duplex optical link 32 is provided from/to the microprocessor 52 via an LED driver 34 and a phototransistor 36. The RF transmitter 30 includes an inphase/quadrature (I/Q) modulator 58, a frequency synthesizer 60 and a power amplifier 62.

In operation, an analogue signal received from the connector electrodes 38 is first amplified by the AMP 44 and then submitted to the LPF 46. The signal is then treated by the MUX 48, and the resulting analogue signal received from the sensor interface module 26 is processed by the A/D 50, as described further below. The A/D 50, for example may be a 12 bit, 500 sample/second/channel analogue to digital converter, although, it is understood that any A/D capable of providing a digital signal for operation in this transmitter device is acceptable. The :C 52 of the digital controller 28 illustrated in Fig. 2 handles mixed signal inputs from a number of sources including analogue signals from the sensor interface 26. These other signals include a digital signal "patient call" button used by the patient for event timing, digital signals for input "lead off', and two digital signals for low power alarms from the battery and a digital pacer detect line referenced to a timer for accurate timing are all collectively indicated as digital inputs 40. Connectors for digital input from other sensor devices such as a pulse oximeter, non invasive blood pressure monitors, patient location, RS232 devices, and others can be included. The analogue channel bandwidth can be 0.1 to 120 Hz at the 3 dB bandwidth points. In the present embodiment the analogue channel bandwidth is 0.1 to 120 Hz.

Features of the transmitter device 14 include its ability to provide as many as 32 channels in this embodiment with groupings of 8,16,24, or 32 or individual 1 through 8 channels, i.e., any combination of between 1 and 32 (with the possibility of as many as 64) channels can be sampled, put in packet format, and transmitted. The A/D input bandwidth is a minimum of 8 kHz. The input noise of a preferred embodiment of this device is 4 µV pk/pk (0.1 to 120 Hz) equivalent input noise. The input differential amplifier 44 and low pass filter 46 of sensor interface/analogue controller 26 have a fixed gain of 2,000 with a maximum input analogue level of 2 mV. Because the gain is fixed, the output of this section has a maximum level of 4 V pk to pk. The maximum level of noise allowed in this amplifier and filter section is 4 mV pk to pk and the maximum differential offset at the input is +/- 300 mV. Because the gain is fixed, no internal calibration is necessary: External calibration can be supplied by replacing the input lead block with a calibrator. The analogue amplifier 44 low pass filter 46 and multiplexer 48 are addressed by the digital controller 28 to select one of 32 channels for input to the A/D 50.

The digital processor/controller 28 first converts the analogue signal in the A/D 50 to a 12 bit digital word. This, along with the digital information from digital inputs 40 (lead disconnects, battery, patient call, etc.), are formatted into a packet with three packet sync bytes, two word sync bytes, packet length, checksum and transmitter number, and are sent through the parallel to serial formatter 56 to the RF transmitter 30. The controller 28 has non-volatile memory for storage of parameter changeable information downloaded through the optical link 32 with the receiver photo transistor 36. Examples of this include packet sync byte value, word sync byte value, frequency of transmission, identification number, number of analogue channels, and modification of the RF serial to I/Q modulator transmitter (TX) functions.

The full duplex optical link provided to the µC 52 by means of a transmission line under control of the LED driver 34, and a receiving line connected to the phototransistor 36 allows the mobile transmitter of an embodiment of the present invention to be reconfigured during operation. That is the characteristics of the scrambling, digital code sequence, frequency and data rate, in addition the frequency of transmission can be changed "on the fly". This unique feature allows the user 24 to make changes while the transmitter is operational, hence significantly enhances the flexibility of the system. The full duplex optical link has the additional advantages of not requiring physical connection between the mobile transmitter and the receiver, providing protection against electrostatic discharge (ESD) and enhancing the ease of use of the system. The operator 24, responsive to operation of the transmitter 14 as displayed on the monitor 22 may place the mobile transmitter 14 adjacent to the receiver 20 so as to align the corresponding optical receivers and transmitters and using a configuration menu on the monitor effect a reconfiguration of the transmitter 14 while the transmitter is operating. The operator 24 is then able to see the affect of the reconfiguration on the monitor 22.

The RF transmitter 30 receives the packetted serial bits then adds a chipping sequence and provides this combined signal to the I/Q modulator 58 within the RF transmitter 30. The data is then transmitted by broad band quadrature phase shift key (DQPSK) synthesized frequency direct sequence spread spectrum signal via antenna 42. This method provides greater transmission rate in the same bandwidth. This method reduces the serial data stream bit rate by half and consequently provides double the data rate within the same RF bandwidth. The DQPSK can, instead of doubling the data rate increase the digital code chipping sequence by double within the same band width thereby providing greater immunity to interference and noise. The output power level of the amplifier 62 of the RF transmitter 30 is 13 dBm.

The use of a frequency synthesizer 60 in the RF transmitter 30 provides multiple frequencies of transmission within, for example, the 902 to 928 MHz band of frequencies. Consequently, as in a preferred embodiment five distinct frequencies are provided.

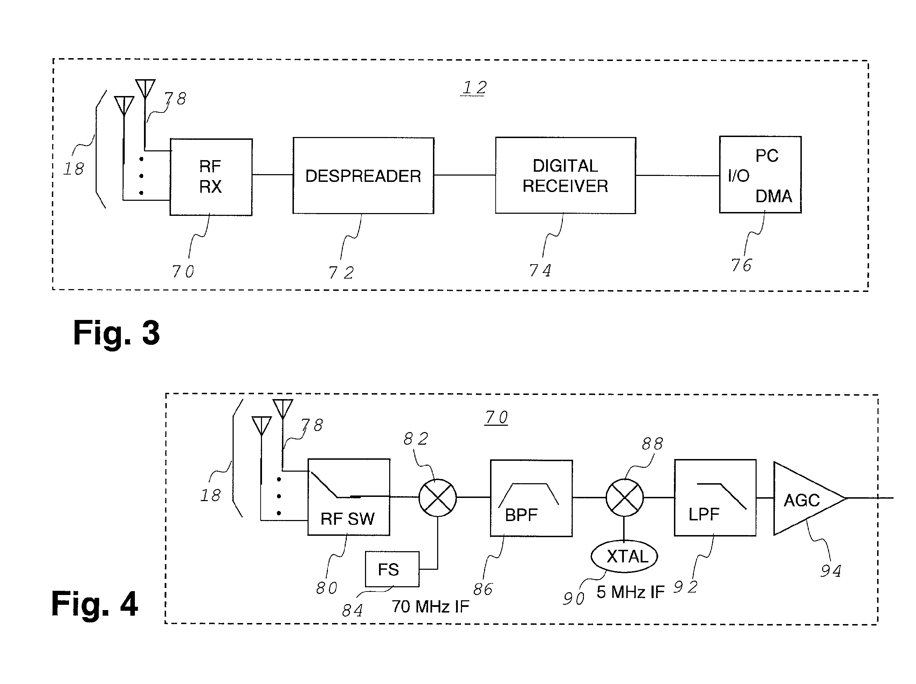

Referring to Fig. 3 there is illustrated in a block diagram, details of the receiver of Fig. 1. The receiver (central monitor interface) 12 includes four functional modules, an RF receiver 70, a despreader 72, a digital receiver/processor 74, and a computer interface direct memory access input/output (DMA I/O) 76. A more detailed view of the receiver is shown in Fig. 4. The radio frequency receiver (RFRX) 70 includes a plurality of antennas 78, a radio frequency switch (RFSW) 80 coupled to the antennas 78, a mixer 82, a frequency synthesizer (FS) 84 for a 70 MHz intermediate frequency (IF), a band pass filter (BPF) 86, a mixer 88 and a crystal (XTAL) 90 for a 5MHz IF low pass filter (LPF) 92 and an automatic gain control (AGC) 94.

In operation, the RF receiver 70 down converts the transmitted RF signal to a 5 MHz second IF and provides this signal to the despreader 72 for demodulation. The antenna system 18 includes two or more antennas 78 to be mounted (under normal conditions) further than 5 feet from the personal computer (PC) 22. The receiver box 20 is to be located within 5 feet of the PC 22. Antenna switches 80 are provided for antenna spacial diversity. An RSSI (received signal strength indicator) signal along with the Bit Error Rate (BER) are provided to allow for switching of the antennae. In a preferred embodiment the AGC 94 O/P will provide a constant level of control over a broad input range. For example, during normal operation, a patient will be mobile (the effect of multipath is prevalent whether mobile or not). The output signal from the RF transmitter 14 will be transmitted in a straight line to the receiver (18/20) and in multiple reflected paths to the receiver. The signals at specific points will tend to cancel each other providing "null" locations where the signal appears to be gone, or disappear. These locations are dependent on many factors, for example, room layout, moving objects and personnel, location and arrangement of adjacent buildings, and metal objects. The frequency width of these nulls are broad in nature and can be several MHz wide. This would degrade the performance of the received signal and reduce the available time to receive the RF. To overcome these problems, spacial diversity of antennas 78 for two or more per receiver with receiver intelligence to switch on poor receive signals is necessary. In a preferred embodiment RSSI is used to switch in antennuators for the input RF signal but uses the packet checksum on the received signal to switch antennas 78. Furthermore, spacial antennas also allow a wider range of travel for a particular patient 16.

The FS 84 used in the RF receiver adjusts the frequencies of the receiver. Before fitting a patient with a transmitter, the transmitter is configured by a PC to provide patient number and to set the frequency of transmission via the full duplex optical link 32. The control signals for the RF receiver FS 84 are also controlled from the PC and can be changed by the operator 24. This provides flexibility along with the ability to change the RF transmitter frequency in situations where it is preferable to avoid consistent interferers within spread spectrum band limitations or provide multiple patients. The PC also provides control over the receiver similar to the transmitter in that the packet and word sync are changeable along with the receiver data structure. The incoming received signal from either antenna 78(controlled by the RF switch 80) is mixed with the RF signal from a frequency synthesizer 84. The 70 MHz IF (intermodulation frequency) is band pass limited to the band pass filter 86 and then goes through a second fixed mixer 88 to provide a second IF of 5 MHz. This signal is low pass filtered by LPF 92 and goes through an AGC circuit 94, which is controlled by the despreader 72 using the RSSI. The 5 MHz IF signal goes to a despreader 72 (Fig. 3) that digitally demodulates the incoming IF and provides a serial data line and a reconstructed clock.

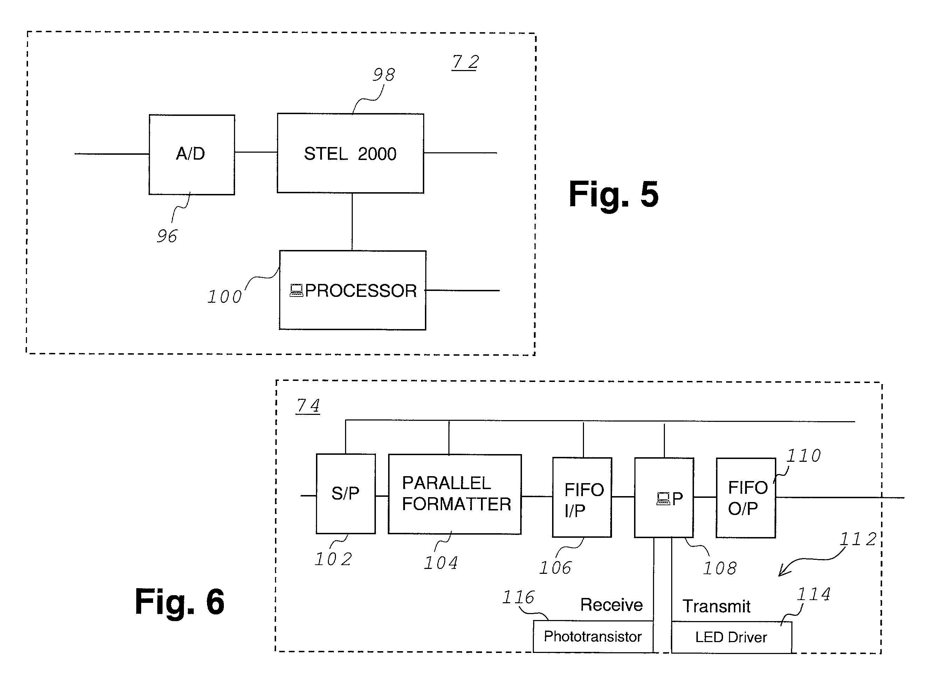

Referring to Fig. 5, there is illustrated in a block diagram, the despreader of Fig. 3 in further detail. The despreader 72 includes an analogue-to-digital converter (A/D) 96, a STEL 2000 98 and a microprocessor 100. The parameters of the elements of a despreader are controllable by the receiver. This allows the chipping sequence, the digital peak detection, and digital filter to be changed, i.e., the frequency of received signal (which is passed on to the RF receiver) and other parameters can be modified to improve the signal quality. From the despreader 72, the serial packet (with spreading signal removed) goes to the digital receiver 74.

Referring to Fig. 6, there is illustrated in a block diagram, the digital receiver of Fig. 3. The digital receiver 74 includes a serial to parallel convertor (S/P) 102, a parallel formatter 104, an input FIFO 106, a microprocessor (µP) 108 and an output FIFO 108. In the digital receiver 74 (the elements of which are shown in greater detail in Fig. 6) the three packet bytes (value controlled by PC) are used to synchronize the input serial data stream. The data is converted to 8-bit byte format using loaded parameters controlled by the PC and stored in the FIFO (first in first out) memory 106. The microprocessor 108 synchronizes the input parallel data from the two word sync bytes defined by the PC, strips this off the parallel data, ensures that the checksum is correct and transfers this to the DMA I/O 76 of the PC 22. Connected to the microprocessor in the receiver is the receiver side 112 of the duplex optical link described above. The transmitter provided by LED Driver 114, the receiver provided by the phototransistor 116.

The PC can also download changes to the packet structure (i.e., one channel, eight channels, thirty-two channels etc.). This data is then displayed in a user appropriate format on the PC.

The computer (central monitoring station) interface 76 is an industry standard digital I/O with DMA (such as the Keithley Metrabyte PDMA-32). The DMA I/O is installed in a personal computer 22 that provides for platform independent high speed data transfer with a minimum of software overhead.

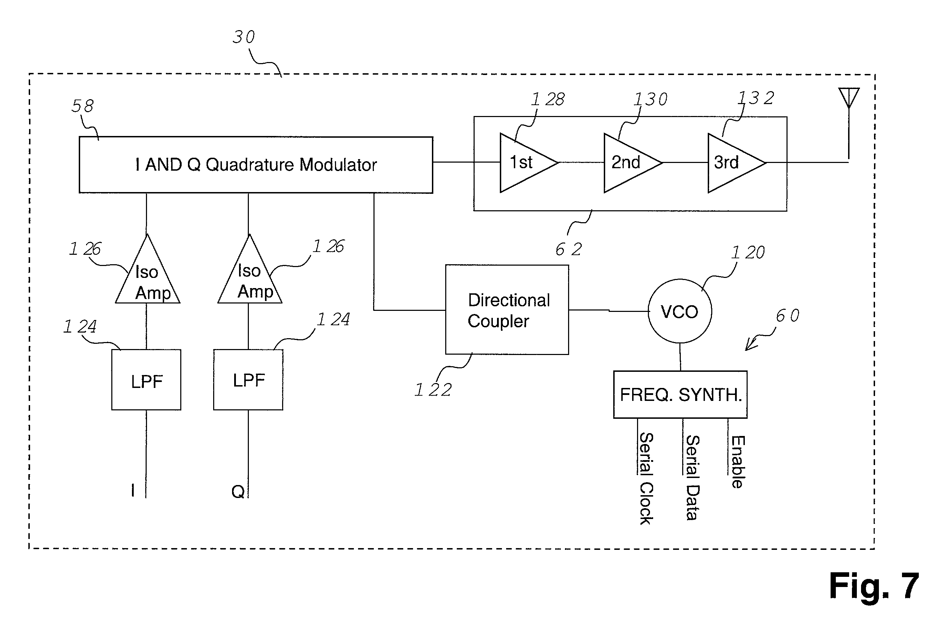

Referring to Fig. 7, there is illustrated, in a block diagram, further detail of the mobile transmitter of Figs. 1 and 2. Specifically further detail of the RF transmitter 30 is shown. The RF transmitter 30 includes the I/Q modulator 58, the frequency synthesizer 60 and the output power amplifier 62. The frequency synthesizer 60 includes a voltage controlled oscillator 120 and a directional coupler 122 and has inputs for serial clock, serial data and enable. The I/Q modulator includes separate low pass filters 124 and isolation amplifiers 126 for the I and Q inputs to the I/Q quadrature modulator. The output power amplifier 62 includes three stages with first 128, second 130 and third 132 stage amplifiers.

While still considering the mobile transmitter 14, to meet the demand of 24 hours between battery changes and to limit the size of the transmitter, a comprehensive design change was undertaken to reduce the current and limit the size. Fig. 7 illustrates the design changes which allowed these objectives to be met. The analogue differential front end (one per channel) is designed to use discrete operating amps to ensure minimum current. No currently designed differential amp could provide such low currents. Lower switching speeds, slower A/D's and low current microcontroller are used to reduce current consumption. The design of the RF amplifier illustrated in Fig. 7 provides +13dBm linear output while using a passive quadrature modulator. In addition, the use of the optical link, rather than adding and RF receiver to effect parameter data transfers saves both complexity and power usage in the mobile transmitter 14.

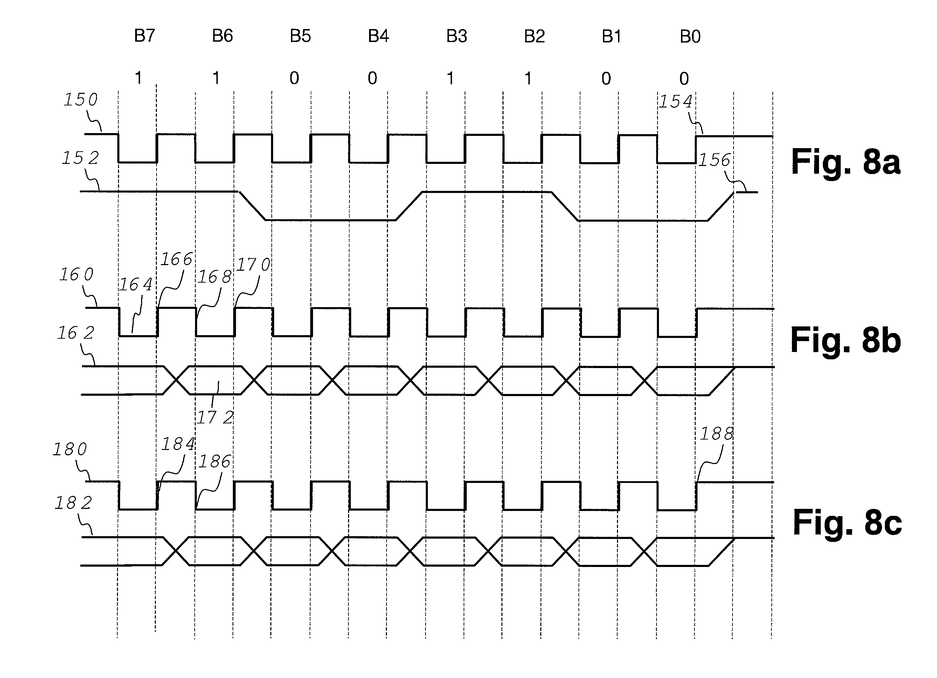

Referring to Figs. 8a, 8b, and 8c there are graphically illustrated optical channel signal formats between the digital receiver 74 and the mobile transmitter 14. Fig. 8a, illustrates the output signals for the mobile transmitter 14 and digital receiver 74 after the digital receiver has been initiated by the computer 22 a signal 150 represents the output optical signal of the mobile transmitter 14 and a signal 152 represents the output optical signal of the digital receiver 74. The mobile transmitter 14 sends a pulse out the optical transmit 34 once every 2 ms period. The pulse duration is between 50 µs and 100 µs, as shown by the signal 150. The digital receiver 74, receives these pulses and outputs a code, which is stable from the end of one transmit pulse to the beginning of the next pulse, as shown by the signal 152. The mobile transmitter 14 receives this signal and shifts the signal until it matches a predefined preamble code. The code for the preamble is 11001100. After transmission the eighth bit of the preamble code, the digital receiver 74 prepares to send the first bit of the first data byte. The mobile transmitter 14 acknowledges a received preamble by holding its signal high for 1 ms, as shown at 154. If the time that the mobile transmitter's optical signal stays high is longer than 100 µs (assuming the preamble is correct) then the digit receiver outputs the first data bit of the command, as indicated at 156.

Fig. 8b graphically illustrates the output signals for the mobile transmitter 14 and the digital receiver 14 during transmission of data from the digital receiver 74 to the mobile transmitter 14. A signal 160 represents the optical signal output by the mobile transmitter 14, and a signal 162 represents the optical signal output by the digital receiver. The signal 160 will go low for 50-100 µs, as indicated at 164 and the data signal 162 from the digital receiver 74 must be stable at that time. When the signal 160 goes high at 166, the bit being sent by the digital receiver can charge, but must be stable by the time the signal 160 goes low at 168. On the rising edge of the signal 160, at 170, the data bit 172 is read at the mobile transmitter 14. On the eighth clock pulse the digital transmitter goes high for 1 ms. At the same time the digital receiver 74 signal 16 goes low for about 500 µs. The digital receiver 74 then sets up the output for the first bit of the second byte and remains stable while the mobile transmitter signal goes low after the 1 ms, then goes high. The signals 160 and 162 are representative of the second byte also. On the last bit of the last byte, the output of the digital receiver 74 goes high and remains so for 500 µs it then goes low for 1ms. This sets up the mobile transmitter to start sending data.

Fig. 8c graphically illustrates the optical signals output by the digital receiver 74 and the mobile transmitter 14 as signals 180 and 182, respectively. The roles are now reversed from the previous two Figs. 8a and 8b. The digital receiver optical output goes low for 1 ms. The data signal 180 sent by the mobile transmitter 14 must be stable when the digital receiver optical signal goes high as shown at 184. During the high period, the data signal 182 may change, but must be stable before the digital receiver optical output signal goes low again as at 186. At the end of the eighth data bit, the digital receiver optical output signal goes high for 1ms, as at 188. The mobile transmitter output optical signal goes low for 500 µs; failure to do so indicates an error and causes the digital receiver 74 to begin transmitting the preamble again.

While the invention has been particularly shown and described with reference to certain embodiments, it will be understood by those skilled in the art that various other changes in form and detail may be made without departing from the spirit and scope of the invention.

## Claims

1. A system for monitoring a patient via physiological data comprising:
a mobile transmitter including a sensor interface for coupling to sensors disposed on the patient for collecting physiological data therefrom, a digital controller having an input for analogue data from the sensor interface, an output for serial digital data derived from analogue data and an optical receiver and transmitter for establishing a bi-direction optical link for receiving mobile transmitter configuration data, and a radio frequency transmitter for radio transmission of the serial digital data in dependence upon stored mobile transmitter configuration data; and
a base station including an antenna array for receiving the wireless transmission from the mobile transmitter, a receiver including an input coupled to the antenna array, an interface having an output for digital data derived from the radio transmission and an input of mobile transmitter configuration data, and an optical receiver and transmitter for establishing a bi-direction optical link for transmitting mobile transmitter configuration data to an adjacent mobile transmitter, and a monitor coupled to the interface for display of the physiological data and for effecting transfer of mobile transmitter configuration data via the bi-directional optical link during operation of the mobile transmitter.

2. A system as claimed in claim 1 wherein the mobile transmitter configuration data includes a packet sync byte value.

3. A system as claimed in claim 1 wherein the mobile transmitter configuration data includes a word sync byte value

4. A system as claimed in claim 1 wherein the mobile transmitter configuration data includes a frequency of transmission.

5. A system as claimed in claim 1 wherein the mobile transmitter configuration data includes an identification number

6. A system as claimed in claim 1 wherein the mobile transmitter configuration data includes a number of analogue channels.

7. A system as claimed in claim 1 wherein the mobile transmitter configuration data includes radio transmitter characteristics

8. A system as claimed in claim 7 wherein radio transmitter characteristics include scrambling parameters.

9. A system as claimed in claim 7 wherein radio transmitter characteristics include digital code sequence.

10. A system as claimed in claim 7 wherein radio transmitter characteristics include transmitter frequency.

11. A system as claimed in claim 7 wherein radio transmitter characteristics include data rate.

12. A system as claimed in claim 7 wherein the radio transmitter uses differential Quadrature phase shift keying modulation.

13. A system as claimed in claim 12 wherein the radio transmitter transmits a direct sequence modulated spread spectrum signal.

14. A system as claimed in claim 13 wherein the signal is transmitted in a radio frequency range of about 902 to 928 MHz.

15. A system as claimed in claim 13 wherein the signal is transmitted in a radio frequency range of about 2.4 to about 2.5 GHz.

16. A system as claimed in claim 13 wherein the signal transmitted in a radio frequency range of about 5.725 to about 5.785 GHz.

17. A mobile transmitter for monitoring a patient via physiological data comprising:
a sensor interface for coupling to sensors disposed on the patient for collecting physiological data therefrom;
a digital controller having an input for analogue data from the sensor interface, an output for serial digital data derived from analogue data and an optical receiver and transmitter for establishing a bi-direction optical link for receiving mobile transmitter configuration data during operation of the mobile transmitter; and
a radio frequency transmitter for radio transmission of the serial digital data in dependence upon stored mobile transmitter configuration data.

18. A base station for monitoring a patient via physiological data comprising:
an antenna array for receiving the wireless transmission from the mobile transmitter;
a receiver including an input coupled to the antenna array, an interface having an output for digital data derived from the radio transmission and an input of mobile transmitter configuration data, and an optical receiver and transmitter for establishing a bi-direction optical link for transmitting mobile transmitter configuration data to an adjacent mobile transmitter; and
a monitor coupled to the interface for display of the physiological data and for effecting transfer of mobile transmitter configuration data via the bi-directional optical link during operation of the mobile transmitter.
